(19) Europäisches Patentamt
European Patent Office
Office européen des brevets

(11) **EP 3 932 487 A1**

(12) **EUROPEAN PATENT APPLICATION**
published in accordance with Art. 153(4) EPC

(43) Date of publication:
**05.01.2022 Bulletin 2022/01**

(21) Application number: **20762309.1**

(22) Date of filing: **12.02.2020**

(51) Int Cl.:
*A61P 9/00* *(2006.01)*          *A61P 35/00* *(2006.01)*
*A61P 43/00* *(2006.01)*          *A61P 17/00* *(2006.01)*
*A61K 31/436* *(2006.01)*         *A61K 31/662* *(2006.01)*

(86) International application number:
**PCT/JP2020/005252**

(87) International publication number:
**WO 2020/175131 (03.09.2020 Gazette 2020/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.02.2019 JP 2019034071**

(71) Applicant: **Osaka University**
**Suita-shi, Osaka 565-0871 (JP)**

(72) Inventor: **KANEDA Mari**
**Suita-shi, Osaka 565-0871 (JP)**

(74) Representative: **TBK**
**Bavariaring 4-6**
**80336 München (DE)**

(54) **EXTERNAL PREPARATION FOR VASCULAR ABNORMALITY TREATMENT**

(57)     To provide a topical preparation capable of treating a vascular anomaly, at least one selected from the group consisting of sirolimus and a sirolimus derivative is percutaneously or transmucosally administered, and thereby vascular anomalies (vascular malformations and vascular tumors) can be treated. Examples of the sirolimus derivative include everolimus, temsirolimus, ridaforolimus, and zotarolimus. A topical preparation containing sirolimus and the sirolimus derivative is excellent in therapeutic effect and safety.

FIG.1

BEFORE EXTERNAL APPLICATION     AFTER 12-WEEK
                                 EXTERNAL APPLICATION

EP 3 932 487 A1

**Description**

Technical Field

The present invention relates to treatment of a vascular anomaly.

Background Art

Vascular anomalies are broadly classified into two groups : vascular tumors and vascular malformations. Causes for most of the vascular tumors and the vascular malformations are unknown, and radical treatment methods therefor have yet to be established. The vascular tumors and the vascular malformations are often commonly called hemangiomas, but are classified into different diseases according to a classification proposed by an international society for medical practice of vascular anomalies, namely the International Society for the Study of Vascular Anomalies (ISSVA). This classification is becoming an international standard, but hitherto commonly used terms are also often used even today. For example, strawberry hemangioma is infantile hemangioma according to the ISSVA classification. In addition, hemangioma simplex, teleangiectasia, a portwine stain, lymphangioma, cavernous hemangioma, venous hemangioma, intramuscular hemangioma, synovial hemangioma, and arterial hemangioma are classified as vascular malformations according to the ISSVA classification.

In general, the most frequent among cases diagnosed as "hemangiomas" is infantile hemangioma, many cases of which spontaneously regress during childhood but leave a scar. Meanwhile, blood vessel malformations do not spontaneously regress, and progression of pathological conditions thereof causes a pain, an ulcer, abnormal growth of an affected limb, a functional disorder, a cosmetic problem, and the like. The vascular malformations are subclassified depending on constituent elements, such as arteries, veins, capillaries, and lymphatic vessels, and combined types thereof also exist. Further, the vascular malformations are often accompanied by anomalies in other organs. The vascular malformations include: a vascular malformation that causes a small lesion and is treatable by resection; and even a refractory vascular malformation that is multiple or huge, infiltrates surrounding tissues, and shows resistance to treatment (Non Patent Literature 1) .

The inventors of the present invention found that a skin lesion involved in tuberous sclerosis complex was treatable by using a topical preparation containing sirolimus (rapamycin), and developed the topical preparation as a pharmaceutical product (Patent Literature 1) . In addition, the inventors found that sirolimus suppressed perspiration (Patent Literature 2) and was usable for treatment of Fabry disease (Patent Literature 3) . Further, the inventors found that neurofibroma was treatable with a topical preparation containing sirolimus (Patent Literature 4).

Citation List

Patent Literature
[PTL 1] WO 2012/105521 A1
[PTL 2] WO 2015/199248 A1
[PTL 3] WO 2017/154675 A1
[PTL 4] WO 2016/152519 A1

Non Patent Literature

[NPL 1] Japanese clinical practice guidelines for vascular anomalies 2017

Summary of Invention

Technical Problem

An obj ect of the present invention is to provide a topical preparation for treating a vascular anomaly.

Solution to Problem

The inventors of the present invention have made extensive investigations in view of the above-mentioned object, and as a result, have found that a topical preparation containing sirolimus and a sirolimus derivative is effective for the treatment of a vascular anomaly. Thus, the inventors have completed the present invention.

1. A topical preparation for treating a vascular anomaly, including as an active ingredient at least one selected from the group consisting of sirolimus and a sirolimus derivative.

2. The topical preparation according to the above-mentioned item 1, wherein the vascular anomaly is a vascular malformation.

3. The topical preparation according to the above-mentioned item 2, wherein the vascular malformation is a blood vessel malformation and/or a disease including a blood vessel malformation as a pathological condition.

4. The topical preparation according to any one of the above-mentioned items 1 to 3, wherein the sirolimus derivative is at least one selected from the group consisting of everolimus, temsirolimus, ridaforolimus, and zotarolimus.

5. The topical preparation according to any one of the above-mentioneditems 1 to 4, wherein the topical preparation is a gel.

6. The topical preparation according to any one of the above-mentioned items 1 to 5, further including an alcohol.

7. The topical preparation according to the above-mentioned item 6, wherein the alcohol is ethanol or isopropanol.

8. The topical preparation according to any one of the above-mentioned items 1 to 4, further including an ethylene glycol ether.

9. The topical preparation according to any one of the above-mentioned items 1 to 8, wherein a concentration of the active ingredient is from 0.04 wt% to 0.8 wt%.

10. A treatment method for a vascular anomaly, including percutaneously administering or transmucosally administering at least one selected from the group consisting of sirolimus and a sirolimus derivative.

11. The treatment method according to the above-mentioned item 10, wherein the percutaneously administering is performed.

Advantageous Effects of Invention

The topical preparation of the present invention exhibits an effect of suppressing the aggravation of the symptoms of the vascular anomaly and an effect of ameliorating the symptoms. The topical preparation of the present invention has, for example, a cell growth-suppressing action, a VEGF production-suppressing action, and an HIF-1$\alpha$ production-suppressing action, and also acts on the nervous system to exhibit an effect of suppressing the growth of vascular endothelial cells, an effect of suppressing the formation of new vessels, and an effect of suppressing vascular ectasia (enlargement of the diameters of blood vessels or lymphatic vessels) . The topical preparation of the present invention exhibits those effects, and hence can reduce hemangioma, suppress the formation of new vessels (in particular, angiogenesis) in vascular malformations, and regress erythema caused by the increase or ectasia of blood vessels or the color tone of a lesion portion.

The topical preparation for treating a vascular anomaly of the present invention is easy to administer and can be administered in a patient's home or the like, and hence does not require frequent hospital visits. Further, the topical preparation can be directly administered to a lesion portion, and can retain the active ingredient at a high concentration at the lesion portion. In addition, even when the active ingredient is present at a high concentration at the lesion portion, the concentration of the active ingredient in blood is kept relatively low so as to enable safe treatment hardly showing an adverse influence on a living body or side effects.

Brief Description of Drawings

FIG. 1 includes photographs for showing the results of administration of a sirolimus-containing gel to two patients in which erythema due to the ectasia of blood vessels or the increase of subepidermal capillaries is observed.

FIG. 2(a) is a graph for showing the cell proliferation-suppressing action of sirolimus on human angiosarcoma-derived vascular endothelial cells. FIG. 2(b) is a graph for showing the cell proliferation-suppressing action of sirolimus on normal human vascular endothelial cells. Sirolimus is effective for the vascular endothelial cells derived from a tumor in which the proliferation of endothelial cells is vigorous as compared to the normal human vascular endothelial cells, and the proliferation of the cells is suppressed in a sirolimus concentration-dependent manner.

FIG. 3 includes micrographs (bright-field imaging) for showing the proliferation state of cells after 48-hour culture following the addition of sirolimus to human angiosarcoma-derived vascular endothelial cells.

FIG. 4 includes micrographs (bright-field imaging) for showing the proliferation state of cells after 96-hour culture following the addition of sirolimus to normal human vascular endothelial cells .

FIG. 5 includes graphs for showing the results of measurement of the protein amounts of VEGF after a lapse of from 24 hours to 72 hours from the addition of sirolimus (20 nM) to (a) human normal fibroblasts, (b) infant-derived normal fibroblasts, (c) HaCaT cells, (d) tuberous sclerosis complex patient-derived angiofibroma cells, and (e) neurofibromatosis type 1patient-derivedneurofibroma cells. The horizontal axis represents time after sirolimus addition. Symbol **"■"** represents 20 nM sirolimus added, and symbol "□" represents a control (no sirolimus added).

FIG. 6 includes photographs of the face of Patient A before the administration of a sirolimus gel and after 12-week administration.

FIG. 7 includes photographs of the penis and scrotum of Patient B before the administration of a sirolimus gel and after 12-week administration, the upper photograph being a front-view photograph, the lower photograph being a photograph taken of the same site from an oblique direction.

FIG. 8 includes photographs of the buttocks of Patient B before the administration of a sirolimus gel and after 12-week administration.

FIG. 9 includes photographs of a sole of Patient B before the administration of a sirolimus gel and after 12-week administration.

FIG. 10 includes photographs of a femoral region of Patient C before the administration of a sirolimus gel and after 12-week administration.

Description of Embodiments

The present invention is not limited to embodiments described below, and various changes may be made thereto within the scope of claims . Embodiments and Examples obtained by combining as appropriate technical means disclosed in different embodiments and Examples are also included in the technical scope of the present invention.

A topical preparation for treating a vascular anomaly according to the present invention contains at least one selected from the group consisting of sirolimus and a sirolimus derivative as an active ingredient.

The sirolimus derivative is not particularly limited, and examples thereof may include everolimus, temsirolimus, ridaforolimus, and zotarolimus. Those sirolimus derivatives are each known to have nearly the same basic structure as the basic structure of sirolimus, and to have physiological activity comparable to that of sirolimus. Therefore, each of those sirolimus derivatives maybe used, as well as sirolimus, as the active ingredient according to one embodiment of the present invention.

Sirolimus is already in use as topical therapeutic drugs for tuberous sclerosis complex and the like, and has been confirmed to be clinically safe. Accordingly, a more preferred active ingredient is sirolimus.

The topical preparation for treating a vascular anomaly according to the present invention may be used for ameliorating various symptoms developed in a patient with a vascular anomaly, suppressing the aggravation of the symptoms, and suppressing the progression of the condition of the disease. The vascular anomaly in the present invention is a disease to be diagnosed on the basis of the " Japanese clinical practice guidelines for vascular anomalies 2017" Examples of the symptoms of the vascular anomaly include a pain, an ulcer, bleeding, abnormal growth of an affected limb, a functional disorder, and a cosmetic problem. Systemic spread of the vascular anomaly leads to anomalies in the state of systemic circulation, thereby causing cardiac hypertrophy, heart failure, or the like and also affecting vital prognosis.

Vascular anomalies include vascular malformations and vascular tumors (hemangiomas), and the topical preparation of the present invention may be used for the treatment of a vascular malformation and/or a vascular tumor (hemangioma). The vascular tumors (hemangiomas) include, for example, infantile hemangioma and angiosarcoma.

The topical preparation of the present invention may be suitably used for the treatment of a vascular malformation. Vascular malformations are classified into four categories: simple vascular malformations; combined vascular malformations; vascular malformations of major named vessels; and vascular malformations associated with other anomalies. The topical preparation of the present invention can treat all vascular malformations included in those categories. The simple vascular malformations include capillary malformation, venous malformation, lymphatic malformation, and arteriovenous malformation. The combined vascular malformations are each a mixture of a plurality of vascular malformations, and examples thereof include various combinations. The vascular malformations of major named vessels include aplasia, origin and course anomalies, hypoplasia, and stenosis/ectasia/aneurysm/shunt of blood vessels or lymphatic vessels having an anatomical name, and persistence of embryonal vessels. The vascular malformations associated with other anomalies include syndromes in which vascular malformations are complicated with anomalies in soft tissues and the skeleton, such as leg length discrepancy and hemihypertrophy. The associated syndromes include, for example, Klippel-Trenaunay-Weber syndrome, Klippel-Trenaunay syndrome, Parkes Weber syndrome, Servelle-Martorell syndrome, Sturge-Weber syndrome, CLOVE syndrome, and Proteus syndrome.

The topical preparation of the present invention may be suitably a topical preparation for treating a blood vessel malformation (capillary malformation, venous malformation, and arteriovenous malformation) and/or a disease including a blood vessel malformation as a pathological condition. Herein, the "disease including a blood vessel malformation as a pathological condition" means a disease that includes a blood vessel malformation, and that is one of the combined vascular malformations, the vascular malformations of major named vessels, and the vascular malformations associated with other anomalies.

Herein, blood vessel malformations include, for example, hemangioma simplex, teleangiectasia, a portwine stain, cavernous hemangioma, venous hemangioma, intramuscular hemangioma, synovial hemangioma, and arterial hemangioma, which are conventional disease names, and lymphatic malformations include, for example, lymphangioma, which is a conventional disease name.

Herein, the "topical preparation for treating" refers to, for example, a topical preparation exhibiting an effect included in the following types.

(1) The development of one or more symptoms associated with a disease is prevented, or the risk of development thereof is reduced.
(2) The recurrence of one or more symptoms associated with a disease is prevented, or the risk of recurrence thereof is reduced.
(3) One or more symptoms associated with a disease are alleviated, or the symptoms are caused to disappear.
(4) The aggravation of one or more symptoms associated with a disease is suppressed, or the progression thereof is suppressed.
(5) The rate of progression of the aggravation of one or more symptoms associated with a disease is reduced.

The topical preparation for treating a vascular anomaly of the present invention may be used for humans and non-human mammals . Examples of the mammals excluding humans include: even-toed ungulates, such as cattle, wild boars, pigs, sheep, and goats; odd-toed ungulates, such as horses; rodents, such as mice, rats, hamsters, and squirrels; Lagomorpha, such as rabbits; and carnivorans, such as dogs, cats, and ferrets. In addition, such non-human animal may be suitably a domestic animal or a companion animal (pet animal).

The topical preparation of the present invention is more preferably percutaneously administered or transmucosally administered, still more preferably percutaneously administered.

The topical preparation of the present invention retains the active ingredient at a high concentration at a lesion portion and allows little thereof to move into blood, and hence is excellent in therapeutic effect and safety.

The present application also relates to the invention of a treatment method for a vascular anomaly including percutaneously administering or transmucosally administering at least one selected from the group consisting of sirolimus and a sirolimus derivative. A more preferred administration method is percutaneous administration. Embodiments of the treatment method for a vascular anomaly are similar to embodiments of the topical preparation for treating a vascular anomaly.

As a dosage form of the topical preparation of the present invention, there are given, for example, a gel, a cream, an ointment, a patch, a poultice, a liniment, and a lotion. The gel and the cream are more preferred dosage forms because the gel and the cream each allow the active ingredient to be easily absorbed into a skin tissue.

The content of the active ingredient in the topical preparation of the present invention is not particularly limited. The following concentration is suitable from the viewpoint of suppressing the moving of the active ingredient into blood and increasing the concentration of the active ingredient at a lesion portion to a therapeutically effective concentration, to thereby suppress systemic side effects and provide a therapeutic effect.

The lower limit of the concentration of the active ingredient may be 0.01 wt% or more, 0.02 wt% or more, 0.03 wt% or more, 0.04 wt% or more, 0.05 wt% or more, 0.06 wt% or more, 0.07 wt% or more, 0.08 wt% or more, 0.09 wt% or more, or 0.1 wt% or more with respect to the total weight of the topical preparation.

The upper limit of the concentration of the active ingredient may be 2.0 wt% or less, 1.5 wt% or less, 1.0 wt% or less, 0.9 wt% or less, 0.8 wt% or less, 0.7 wt% or less, 0.6 wt% or less, 0.5 wt% or less, 0.4 wt% or less, 0.3 wt% or less, 0.25 wt% or less, or 0.2 wt% or less with respect to the total weight of the topical preparation.

The concentration of the active ingredient is, for example, preferably from 0.01 wt% to 2.0 wt%, more preferably from 0.03 wt% to 1.0 wt%, still more preferably from 0.04 wt% to 0.8 wt%, even more preferably from 0.05 wt% to 0.4 wt% with respect to the total weight of the topical preparation.

The daily dose of sirolimus or the sirolimus derivative per unit surface area of a living body is not particularly limited.

For example, the lower limit of the daily dose may be $0.0001 \text{ mg/cm}^2$ or more, $0.0002 \text{ mg/cm}^2$ or more, $0.0005 \text{ mg/cm}^2$ or more, $0.001 \text{ mg/cm}^2$ or more, $0.002 \text{ mg/cm}^2$ or more, or $0.003 \text{ mg/cm}^2$ or more.

For example, the upper limit of the daily dose may be $2 \text{ mg/cm}^2$ or less, $1 \text{ mg/cm}^2$ or less, $0.5 \text{ mg/cm}^2$ or less, $0.05 \text{ mg/cm}^2$ or less, $0.04 \text{ mg/cm}^2$ or less, or $0.03 \text{ mg/cm}^2$ or less.

The dose is, for example, from $0.0001 \text{ mg/cm}^2$ to $2 \text{ mg/cm}^2$, preferably from $0.0002 \text{ mg/cm}^2$ to $1 \text{ mg/cm}^2$, more preferably from $0.0005 \text{ mg/cm}^2$ to $0.5 \text{ mg/cm}^2$, still more preferably from $0.001 \text{ mg/cm}^2$ to $0.05 \text{ mg/cm}^2$. It is appropriate that the above-mentioned amount of sirolimus or the sirolimus derivative be administered daily in a single dose or a plurality of divided doses by application or the like. In other words, the topical preparation according to one embodiment of the present invention is preferably capable of achieving the above-mentioned dose.

A method of preparing the topical preparation is exemplified below. (i) A gel may be prepared by allowing a solution containing sirolimus or the sirolimus derivative to gelate. (ii) An ointment may be prepared by mixing an ointment base with sirolimus or the sirolimus derivative. (iii) Apatch, a poultice, a liniment, a lotion, and a cream may each be prepared in accordance with a well-known method.

The gel, the ointment, and the cream are specifically described below.

(1) Gel

The topical preparation for treating a vascular anomaly of the present invention may be a gel containing sirolimus or the sirolimus derivative.

When a solution containing sirolimus or the sirolimus derivative is allowed to gelate, it is appropriate that the solution be allowed to gelate using a gelation inducer. Examples of the gelation inducer include a carboxyvinyl polymer, carboxymethyl cellulose, aluminum hydroxide, and bentonite.

A specific configuration of the carboxyvinyl polymer is not particularly limited, and Carbopol (trademark), HIVISWAKO (trademark), or AQUPEC (trademark) may be used. Of those, Carbopol (trademark) 934P NF or Carbopol (trademark) 980 is preferred from the viewpoint of good feeling in the case of application as a topical preparation.

For example, when Carbopol (trademark) is used, first, Carbopol (trademark) is added to a solution containing sirolimus or the sirolimus derivative. Further, the pH of the solution is adjusted to be neutral by adding a pH adjuster (e.g., tris(hydroxymethyl)aminomethane or triethanolamine). Thus, the gelation of the solution may be induced.

The gel according to one embodiment of the present invention suitably contains an alcohol. The incorporation of the alcohol can allow the active ingredient contained in the gel to be efficiently absorbed into a skin tissue to reach an affected site. Examples of the alcohol may include ethanol and isopropanol. From the viewpoint of allowing the active ingredient to be more efficiently absorbed into a skin tissue, ethanol is preferred.

The amount of the alcohol is not particularly limited, and only needs to be such an amount that sirolimus or the sirolimus derivative can be sufficiently dissolved. For example, the weight of the alcohol may be from 100 to 300 times as great as the weight of sirolimus or the sirolimus derivative, or may be from 120 to 250 times as great. With respect to the total weight of the topical preparation, the amount of the alcohol is preferably 20 wt% or more, more preferably 30 wt% or more, still more preferably 40 wt% or more. When the amount of the alcohol is 60 wt% or less, excessive evaporation of the alcohol from the formulation is prevented, and hence a formulation having a stable active ingredient concentration can be preserved (stored). Therefore, the amount of the alcohol is more preferably about 50 wt% (from 45% to 55%).

When the alcohol is contained in such an amount that the active ingredient is sufficiently dissolved, the active ingredient can be allowed to be more efficiently absorbed into a skin tissue. As long as the amount of the alcohol is 50 wt% or less, as the amount of the alcohol increases, the active ingredient is more sufficiently dissolved, and hence can be allowed to be more efficiently absorbed into the skin tissue.

The amount of a gelling agent contained in the gel is not particularly limited, and only needs to be an amount sufficient for the solution containing sirolimus or the sirolimus derivative to gelate. The amount of the gelling agent contained in the gel may be, for example, 1.5 wt% or more with respect to the total weight of the gel. More specifically, the amount may be from 1.5 wt% to 20 wt%, from 1.5 wt% to 15 wt%, from 1.5 wt% to 10 wt%, from 1.5 wt% to 5 wt%, or from 1.5 wt% to 2.5 wt% with respect to the total weight of the gel.

The amount of the pH adjuster (neutralizer) contained in the gel is not particularly limited, and may be appropriately set depending on the amounts of the solvent and the gelling agent. The amount of the pH adjuster contained in the gel may be, for example, from 0.5 wt% to 5.0 wt%, from 0.5 wt% to 2.5 wt%, or from 0.5 wt% to 1.0 wt% with respect to the total weight of the gel.

For example, when the gelation inducer (e.g., Carbopol (trademark)) and the pH adjuster (e.g., tris(hydroxymethyl)aminomethane) are used as components for inducing gelation, the amount of the gelation inducer may be, for example, 1.6 wt% with respect to the total weight of the topical preparation, and the amount of the pH adjuster may be, for example, 0.4 wt%, 0.6 wt%, or 0.8 wt% with respect to the total weight of the topical preparation. Of course, the present invention is not limited to the above-mentioned ratios.

The gel may contain a component other than the above-mentioned active ingredient, solvent (alcohol), gelling agent, and pH adjuster (neutralizer). Examples of the other component include a water-soluble polymer, water, and a medicinal component other than sirolimus or the sirolimus derivative.

Examples of the water-soluble polymer include polyethylene glycol, starch, methylcellulose, hydroxypropyl cellulose (HPC), polyvinyl alcohol, polyvinyl methyl ether, and polyvinylpyrrolidone.

The amount of the other component contained in the gel is not particularly limited, but may be, for example, 50 wt% or less, 40 wt% or less, 30 wt% or less, 20 wt% or less, or 10 wt% or less with respect to the total weight of the gel.

The amount of sirolimus or the sirolimus derivative contained in the gel is not particularly limited. However, the amount described above is suitable from the viewpoint of achieving both of the following effects: an effect of suppressing the moving of the active ingredient into blood to prevent systemic side effects; and an effect of increasing the concentration of the active ingredient in a skin tissue to a therapeutically effective concentration to provide a therapeutic effect.

It is appropriate that the gel be applied daily or once every 2 to 3 days. The gel is preferably applied daily. When applied daily, the gel is prefer ably applied 1 to 3 times a day, more preferably applied 2 to 3 times a day, most preferably applied twice a day.

In addition, a suitable active ingredient concentration in the formulation and a suitable frequency of the administration thereof are preferably adjusted depending on, for example, age, an administration site, and a skin thickness. For example, to a young child's thin skin, armpit, or the like, the gel having an active ingredient concentration of from 0.05 wt% to 0.2 wt% may be applied 1 to 3 times a day. In addition, to a palm, a sole, or the like, the gel having an active ingredient concentration of from 0.2 wt% to 0.8 wt% may be applied once or twice a day.

A more specific example of the composition of the gel is described below, but the present invention is not limited to the following composition. With the following configuration, the therapeutic effect of sirolimus or the sirolimus derivative on the vascular anomaly can be further enhanced. In addition, with the following configuration, a desired effect can be obtained with a smaller amount of sirolimus or the sirolimus derivative, and hence the adverse influence thereof on a living body can be alleviated.

The gel may contain Carbopol (trademark) 934P NF, water, an alcohol (e.g., ethanol or isopropanol, preferably ethanol), and tris(hydroxymethyl)aminomethane, in addition to sirolimus or the sirolimus derivative.

In this case, a ratio among the weights of sirolimus or the sirolimus derivative, Carbopol (trademark) 934P NF, water, the alcohol, and tris (hydroxymethyl) aminomethane may be as follows : sirolimus or sirolimus derivative:Carbopol (trademark) 934P NF:water:alcohol:tris(hydroxymethyl)aminomethane=(0.5 to 2) :16:490: (450 to 500):6.

The above-mentioned ratio may be as follows: sirolimus or sirolimus derivative:Carbopol (trademark) 934P NF:water:alcohol:tris(hydroxymethyl)aminomethane=(0.5 to 2):16:490:(480 to 490):6, or may be as follows: sirolimus or sirolimus derivative:Carbopol (trademark) 934P NF:water:alcohol:tris(hydroxymethyl)aminomethane=2:16:490:486: 6.

(2) Ointment

The topical preparation for treating a vascular anomaly of the present invention may be an ointment containing sirolimus or the sirolimus derivative.

As a base, there are given, for example, waxes (e.g., natural waxes, such as white beeswax, lanolin, carnauba wax, and spermaceti; mineral waxes, such as montan wax; and synthetic waxes), paraffins (e.g., liquid paraffin and solid paraffin), and petrolatum (e.g., white petrolatum and yellow petrolatum).

The amount of the base is not particularly limited, but may be, for example, 10 wt% or more, 20 wt% or more, 30 wt% or more, 40 wt% or more, 50 wt% or more, 60 wt% or more, 70 wt% or more, 80 wt% or more, or 90 wt% or more with respect to the total weight of the ointment.

The ointment may contain a component other than sirolimus or the sirolimus derivative. Examples of the other component include the components described in the foregoing section for the gel.

The ointment may contain propylene carbonate, solid paraffin, and white petrolatum in addition to sirolimus or the sirolimus derivative. In addition, the ointment may further contain liquid paraffin in addition to propylene carbonate, solid paraffin, and white petrolatum. In addition, the ointment may further contain white beeswax in addition to propylene carbonate, solid paraffin, white petrolatum, and liquid paraffin.

In this case, a ratio among the weights of sirolimus or the sirolimus derivative, propylene carbonate, solid paraffin, white petrolatum, liquid paraffin, and white beeswax may be as follows: sirolimus or sirolimus derivative:propylene carbonate:solid paraffin:white petrolatum:liquid paraffin:white beeswax=(0.3 to 10) : (50 to 59.4) : (30 to 45) : (895) : (0 to 10) : (0 to 5) . In the above-mentioned ratio, the total of the active ingredient, propylene carbonate, solid paraffin, and liquid paraffin is 105.

More specifically, the above-mentioned ratio may be the following ratio 1, 2, or 3.

(Ratio 1) Sirolimus or sirolimus derivative:propylene carbonate:solid paraffin:white petrolatum:liquid paraffin:white beeswax=2:58:30:895:10:5

(Ratio 2) Sirolimus or sirolimus derivative:propylene carbonate:solid paraffin:white petrolatum:liquid paraffin:white beeswax=2:58:45:895:0:0

(Ratio 3) Sirolimus or sirolimus derivative:propylene carbonate:solid paraffin:white petrolatum:liquid paraffin:white beeswax=2:58:35:895:10:0

When the above-mentioned ratio 1 and ratio 2 are compared to each other, the topical preparation produced at the ratio 1 has higher transparency than the topical preparation produced at the ratio 2, and can be reduced in amount of water present on the surface of the topical preparation.

When the above-mentioned ratio 1 and ratio 3 are compared to each other, the topical preparation produced at the ratio 1 is smoother than the topical preparation produced at the ratio 3, and can be reduced in amount of water present on the surface of the topical preparation.

The amount of sirolimus or the sirolimus derivative contained in the ointment is not particularly limited. However, the amount described above is suitable from the viewpoint of achieving both of the following effects: an effect of suppressing the moving of the active ingredient into blood to prevent systemic side effects; and an effect of increasing the concentration of the active ingredient in a skin tissue to a therapeutically effective concentration to provide a therapeutic effect.

It is appropriate that the ointment be applied daily or once every 2 to 3 days. The ointment is preferably applied daily. When applied daily, the ointment is preferably applied 1 to 3 times a day, more preferably applied 2 to 3 times a day, most preferably applied twice a day.

In addition, a suitable active ingredient concentration in the formulation and a suitable frequency of the administration thereof are preferably adjusted depending on, for example, age, an administration site, and a skin thickness. For example, to a young child's thin skin, armpit, or the like, the ointment having an active ingredient concentration of from 0.05% to 0.2% may be applied 1 to 3 times a day. In addition, to a palm, a sole, or the like, the ointment having an active ingredient concentration of from 0.2% to 0.8% may be applied once or twice a day.

The ointment may be produced in accordance with a well-known method. An example of the production method is described below.

For example, the ointment may be prepared using a homomixer (for example, manufactured by Primix Corporation) or a universal mixer (for example, manufactured by Dalton Corporation) . When the base is solid at room temperature, it is appropriate that the base be heated until becoming liquid, and the base in a liquid state be mixed with a solution having the active ingredient dissolved therein. More specifically, various components that are solid at room temperature (e.g., waxes, paraffins, and petrolatum) are heated to a temperature equal to or higher than their melting point (e.g., 70°C) to be dissolved, a solution having the active ingredient dissolved therein is added to the dissolved matter, and the whole is stirred. After that, while being stirred, the mixture is cooled to around room temperature (e.g., 40°C). Thus, the ointment may be produced.

Another method is as described below. The base is completely dissolved at from 70°C to 80°C, and stirred using a planetary centrifugal mixer (manufactured by Thinky Corporation), in a stirring mode, at 800 rpm for 30 minutes, then at 1,000 rpm for 5 minutes, and then at 2,000 rpm for 1 minute (15°C). After that, to the base, a solution having the active ingredient dissolved therein is added, and the whole is further stirred at 1,000 rpm for 1 minute and then at 2,000 rpm for 1 minute (without cooling) . Thus, an ointment of better quality having dispersed therein extremely fine particles each containing the active ingredient may be prepared.

When the above-mentioned other component is to be incorporated into the ointment, it is appropriate that the ointment be prepared by: preparing a solution in which the active ingredient and the other component are dissolved in a desired solvent; adding the base to the solution; and performing the steps after the addition in accordance with the above-mentioned method.

(3) Cream

The topical preparation for treating a vascular anomaly of the present invention may be a cream containing sirolimus or the sirolimus derivative.

A base and an additive are not particularly limited. As a preferred cream that allows little of sirolimus or the sirolimus derivative to move into blood, there is given a cream containing an ethylene glycol ether.

A diethylene glycol ether is preferably, for example, a diethylene glycol monoalkyl ether. An alkyl group of the diethylene glycol monoalkyl ether is preferably, for example, a $C_1$ to $C_6$ alkyl group. The diethylene glycol monoalkyl ether is preferably any of diethylene glycol monomethyl ether, diethylene glycol monoethyl ether, diethylene glycol monopropyl ether, and mixtures thereof, more preferably any of diethylene glycol monomethyl ether, diethylene glycol monoethyl ether, and mixtures thereof, most preferably diethylene glycol monoethyl ether (2-(2-ethoxyethoxy)ethanol, TRANSCUTOL (trademark) P).

Sirolimus or the sirolimus derivative is soluble in the diethylene glycol ether. Sirolimus dissolves well in the diethylene glycol monoethyl ether, and can be dissolved therein at up to 2.02 w/v%.

Sirolimus or the sirolimus derivative may be dissolved in the diethylene glycol ether before being formulated. The formulation using the diethylene glycol ether helps sirolimus or the sirolimus derivative to penetrate skin, and can retain the active ingredient in a skin tissue. A formulation using diethylene glycol monoethyl ether is particularly excellent in ability to help the active ingredient to penetrate the skin and in ability to retain the active ingredient in the skin tissue.

The content of the diethylene glycol ether in the cream only needs to be such an amount that sirolimus or the sirolimus derivative can be dissolved in the formulation. The content of the diethylene glycol ether is, for example, preferably from 1 wt% to 40 wt%, more preferably from 2 wt% to 30 wt%, still more preferably from 5 wt% to 25 wt%, even more preferably from 10 wt% to 20 wt% with respect to the total weight of the topical preparation.

The base of the cream contains an oily component, an aqueous component, and a surfactant. It may further contain any other additives.

Examples of the oily component include hydrocarbons, fatty acid esters, waxes, higher fatty acids, and higher alcohols. Of those, a hydrocarbon and a higher alcohol are preferred.

Examples of the hydrocarbon include white petrolatum and liquid paraffin. Of those, white petrolatum is preferred. Examples of the higher alcohol include stearyl alcohol, cetanol, myristyl alcohol, lauryl alcohol, and oleyl alcohol. Of those, stearyl alcohol is preferred. An example of the fatty acid ester is isopropyl myristate. Examples of the wax include beeswax and lanolin. An example of the higher fatty acid is stearic acid.

The content of the oily component is, for example, preferably from 20% to 60%, more preferably from 30% to 50% with respect to the total weight of the topical preparation.

Examples of the aqueous component contained in the cream include water, a polyhydric alcohol, and a lower alcohol. Of those, water and a polyhydric alcohol are preferred.

Examples of the polyhydric alcohol include glycerin, propylene glycol, and 1,3-butylene glycol. Of those, propylene glycol is preferred. Examples of the lower alcohol include ethanol and isopropanol.

The content of the aqueous component including water in the cream is, for example, preferably from 20% to 60%, more preferably from 30% to 50% with respect to the total weight of the topical preparation.

The surfactant (emulsifier) is not particularly limited, and a surfactant (emulsifier) well known to a person skilled in the art may be used. Preferred examples of the surfactant (emulsifier) may include polyoxyethylene hydrogenated castor oil 60 and glyceryl monostearate. The content of the surfactant is, for example, preferably from 1% to 10%, more preferably from 2% to 8% with respect to the total weight of the topical preparation.

Examples of the other additives may include a preservative, an antioxidant, and a pH adjuster. In addition, the cream may also contain a medicinal component different from sirolimus or the sirolimus derivative.

The composition of the cream is preferably, for example, as follows with respect to the total weight of the topical preparation: 0.01 wt% to 2.0 wt% of sirolimus or the sirolimus derivative, 1 wt% to 40 wt% of the diethylene glycol ether, 5 wt% to 40 wt% of a hydrocarbon, 5 wt% to 30 wt% of a higher alcohol, 1 wt% to 30 wt% of a polyhydric alcohol, and 10 wt% to 50 wt% of water.

The composition of the cream is more preferably, for example, as follows with respect to the total weight of the topical preparation: 0.04 wt% to 0.8 wt% of sirolimus or the sirolimus derivative, 2 wt% to 30 wt% of the diethylene glycol ether, 10 wt% to 30 wt% of a hydrocarbon, 10 wt% to 25 wt% of a higher alcohol, 3 wt% to 20 wt% of a polyhydric alcohol, and 20 wt% to 40 wt% of water.

The cream is administered by, for example, being applied to an affected site daily or once every 2 to 3 days. The cream is preferably applied daily, more preferably applied 1 to 3 times a day, still more preferably applied 2 to 3 times a day.

In addition, the concentration of the active ingredient in the topical preparation and the frequency of the administration thereof are preferably adjusted depending on, for example, age, an administration site, and a skin thickness. For example, to a young child's thin skin, armpit, or the like, the topical preparation having an active ingredient concentration of from 0.05% to 0.2% may be applied 1 to 3 times a day. In addition, to a palm, a sole, or the like, the topical preparation having an active ingredient concentration of from 0.2% to 0.8% may be applied once or twice a day.

The cream may be produced in accordance with a well-known method. For example, a cream of good quality containing the active ingredient may be prepared by sequentially dissolving and mixing all raw materials for the base at from 70°C to 80°C, emulsifying the materials, then cooling the emulsified materials while mixing the materials, and mixing the cooled product with a solution of the active ingredient. The use of a planetary centrifugal mixer (manufactured by Thinky Corporation) for the emulsification and mixing provides good efficiency.

The present invention is more specifically described below by way of Examples, but the present invention is not limited thereto.

Examples

[Production Examples: Preparation of Sirolimus-containing Gels]

Gels containing sirolimus at various concentrations were prepared. A specific method is as described below. Sirolimus was added to and dissolved in ethanol, and then water for injection was further added and mixed to prepare a mixed solution. Carbopol (trademark) 934P NF was added to and mixed with the mixed solution to prepare a homogeneous suspension. Tris(hydroxymethyl)aminomethane serving as a neutralizer was added to and mixed with the suspension to prepare each of the gels.

Components other than sirolimus in 1 g of each of the gels were 16 mg of Carbopol (trademark) 934P NF, 490 mg of water, 480 mg to 490 mg of ethanol, and 6 mg of tris(hydroxymethyl)aminomethane.

More specifically, the gels were prepared according to the compositions of the following Production Examples 1 to 5.

Production Example 1: A gel containing 0.4 wt% of sirolimus (total amount: 100 g)

| | |
|---|---|
| Sirolimus | 0.4 g |
| Ethanol | 48.4 g |
| Water for injection | 49 g |
| Carbopol (trademark) 934P NF | 1.6 g |
| Tris(hydroxymethyl)aminomethane | 0.6 g |

Production Example 2: A gel containing 0.8 wt% of sirolimus (total amount: 100 g)

| | |
|---|---|
| Sirolimus | 0.8 g |
| Ethanol | 48 g |
| Water for injection | 49 g |
| Carbopol (trademark) 934P NF | 1.6 g |
| Tris(hydroxymethyl)aminomethane | 0.6 g |

Production Example 3: A gel containing 0.2 wt% of sirolimus (total amount: 100 g)

| | |
|---|---|
| Sirolimus | 0.2 g |
| Ethanol | 48.6 g |
| Water for injection | 49 g |
| Carbopol (trademark) 934P NF | 1.6 g |
| Tris(hydroxymethyl)aminomethane | 0.6 g |

Production Examples 4 and 5: A gel containing 0.05% of sirolimus (Production Example 4) and a gel containing 0.1% of sirolimus (Production Example 5) were similarly produced.

[Production Example: Preparation of Sirolimus-containing Cream]

Production Example 6: A cream containing 0.2 wt% of sirolimus (total amount: 100 g)

Sirolimus powder was obtained from Fujian Kerui Pharmaceutical Co., Ltd. The sirolimus powder was added to and dissolved in diethylene glycol monoethyl ether (TRANSCUTOL (trademark) P), and then the solution was mixed with a preliminarily mixed and prepared base (oil-in-water (O/W) emulsion) to yield a cream. Specific composition is shown below.

| | |
|---|---|
| Sirolimus | 0.2 g |
| Diethylene glycol monoethyl ether | 10 g |
| Hydrophilic cream | 89.8 g |
| White petrolatum | 22.45 g |
| Stearyl alcohol | 17.96 g |
| Propylene glycol | 10.78 g |
| Polyoxyethylene hydrogenated castor oil 60 | 3.59 g |
| Glyceryl monostearate | 0.90 g |
| Methyl p-benzoate | 0.09 g |
| Propyl p-benzoate | 0.09 g |
| Purified water | 33.94 g |

[Test Example 1: Erythema Regression]

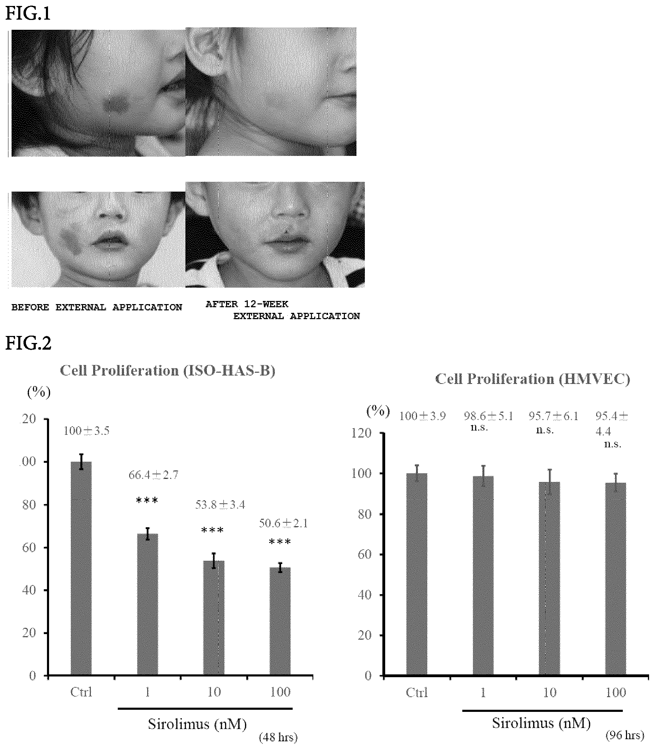

The 0.2% sirolimus-containing gel produced in Production Example 3 was applied to two patients, in which erythema was observed, twice a day for 12 weeks. As a result, the ectasia and construction of blood vessels were suppressed, and the regression of erythema was found (FIG. 1).

[Test Example 2: Cell Proliferation Suppression]

1. Cell Culture

Human angiosarcoma cells (ISO-HAS-B) were obtained from the Cell Resource Center for Biomedical Research, Cell Bank, Tohoku University, and cultured in DMEM (high glucose) supplemented with 10% of fetal calf serum (FCS) under an environment of 37°C and 5% $CO_2$. Normal human vascular endothelial cells (HMVEC-d) were obtained from Lonza Japan Ltd., and cultured in EBM (trademark)-2 medium supplemented with EGM-2 MV under an environment of 37°C and 5% $CO_2$.

2. Cell Proliferation Analysis

a) Analysis based on Cell Count

ISO-HAS-B and HMVEC-d ($1 \times 10^4$ cells/well) were cultured in a 96-well tissue culture plate. Sirolimus was added to medium at a final concentration of 1 nM, 10 nM, or 100 nM. After the addition of sirolimus, ISO-HAS-B and HMVEC-d were cultured for 48 hours and 96 hours, respectively, and then the cells were washed 3 times with PBS and a cell count was measured to evaluate cell proliferation. For the measurement of the cell count, an OD450 was measured in a microplate reader (Model 550; Bio-Rad Laboratories, Inc., Hercules, CA, USA) using a cell counting kitMTT Cell Count Kit (Nacalai Tesque, Inc.). Cell proliferation was quantified in the following manner.

$$T/C \times 100$$

(T: average absorbance of sirolimus-treated group, C: absorbance of control group)

b) Analysis based on Image

ISO-HAS-B and HMVEC-d ($1 \times 10^4$ cells/well) were cultured in a 6-well tissue culture plate. After having been cultured in the same manner as in the foregoing section 1, the cells were washed 3 times with warmed PBS, and morphological images of the cells were obtained by a bright-field observation method (bright-field microscopy).

3. Results

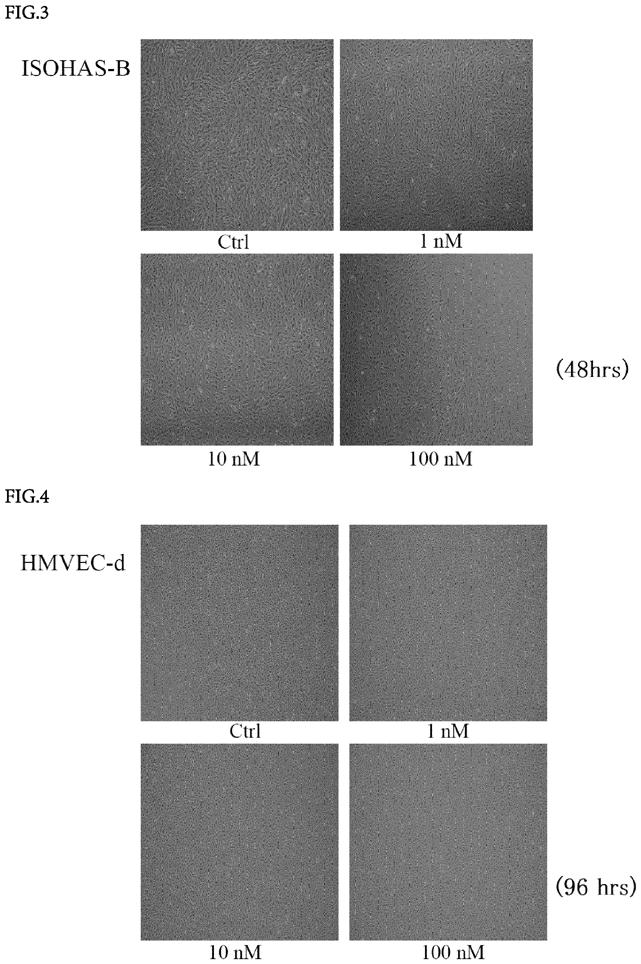

The results are shown in FIGS. 2, FIG. 3, and FIG. 4. The proliferation of the normal human vascular endothelial cells (HMVEC-d) was slightly suppressed by sirolimus, but the proliferation-suppressing effect of sirolimus on the vascular endothelial cells derived from a tumor (ISO-HAS-B) in which the proliferation of endothelial cells was vigorous was high.

[Test Example 3: Suppression of Production of Vascular Endothelial Growth Factor (VEGF)]

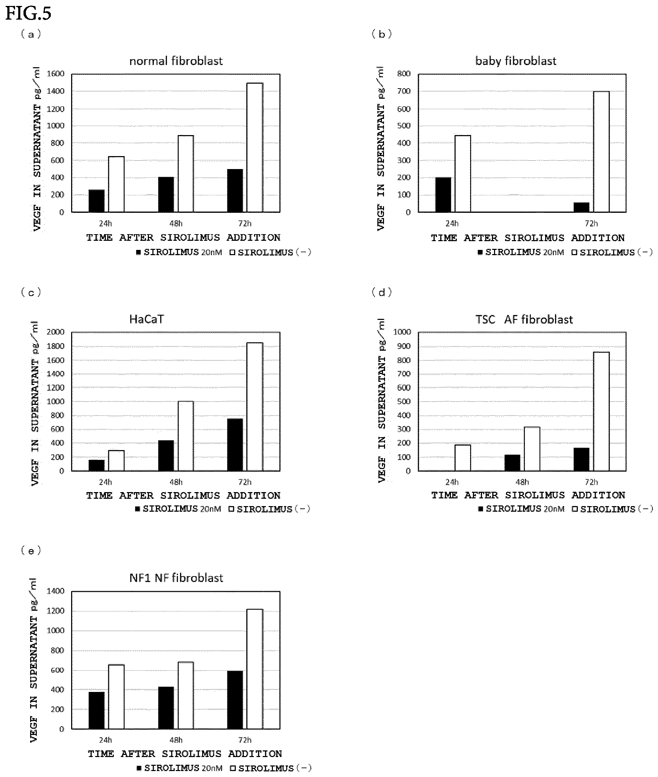

HaCaT cells (human keratinocyte cell line) were purchased from the German Cancer Research Center (dkfz). Human normal fibroblasts, infant-derived normal fibroblasts, tuberous sclerosis complex patient-derived angiofibroma cells, and neurofibromatosis type 1 patient-derived neurofibroma cells were derived from affected tissues resected from patients, and were isolated from resected cells in accordance with a general primary culture method for fibroblasts (edited by Toshio Kuroki et al.: New Cultured Cell Experimental Methods in Molecular Biology Research, Jikken Igaku separate volume, BioManual UP Series Revised 2nd Edition, Yodosha Co., Ltd.). The cells obtained from each of the patients were used with the consent of the patient or guardian. Those cells were cultured using DMEM under an environment of 37 °C and 5% $CO_2$. During the culture of those cells, sirolimus was added to the medium at a final concentration of 20 nM. After 24 hours, 48 hours, and 72 hours of culture from the addition of sirolimus, the culture supernatant was collected and the concentration of VEGF in the culture supernatant was measured using a kit for detecting VEGF (Human VEGF Quantikine (trademark) ELISA Kit, manufactured by R&D Systems). The results are shown in FIG. 5. It was shown that sirolimus suppressed the production of VEGF.

[Test Example 4: Clinical Test]

1. Subjects

The following three patients, who had been diagnosed with vascular anomalies based on Japanese clinical practice guidelines for vascular anomalies 2017, and who had been judged to have no other effective treatments, were subjected to the test. This test was carried out with an approval obtained as a result of deliberation by Ethical Review Board of Osaka University.

Patient A, 9-year-old male, disease: phakomatosis pigmentovascularis 3b Mental retardation is found as well as hemangioma simplex, nevus spilus, and the like. Laser treatment had been tried in the past but had not led to complete cure. Hemangioma simplex is classified as capillary malformation according to the ISSVA classification.

Patient B, 4-year-old male, disease: Blue rubber bleb nevus syndrome (characterized by a venous malformation)

Prominence of dark purple blood vessels ranging from the penis to the scrotum was found. Prominence of greenish purple blood vessels was found on the buttocks, on the abdomen, and from the lower legs to the feet. Pain appeared in the lower legs particularly in the afternoon, but an analgesic had no effect.

Patient C, 23-year-old male, disease: A-V malformation (arteriovenous malformation) For lesions present in the femoral regions, sclerotherapy and laser treatment had been tried in the past but had not led to complete cure, and induration and pain in persistent hemangioma in a deep site, and bleeding from persistent hemangioma on the skin surface had been repeated.

2. Treatment Method

The gel containing 0.2 wt% of sirolimus produced in Production Example 3 (hereinafter referred to as 0.2 % sirolimus gel) was applied to a lesion portion twice a day for 12 weeks. An approximate size of an application target lesion was measured to determine a single application amount. The application amount was set to about 125 mg of gel per 50 cm$^2$. In the sirolimus gel treatment period (12 weeks), no combined medicine was used, and no combination therapy was performed.

3. Evaluation

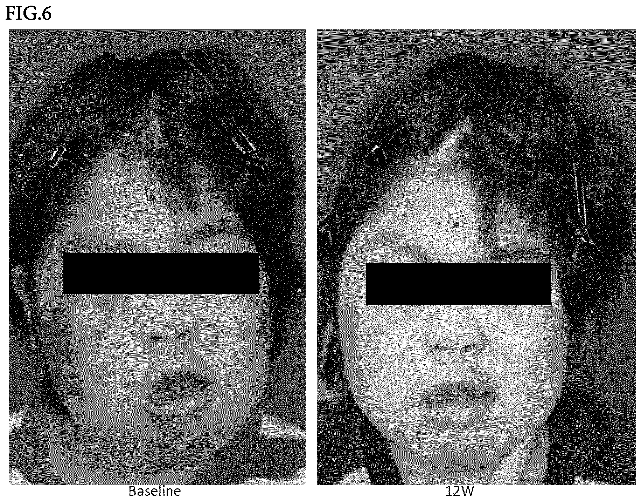

The degrees of amelioration of lesions after 12-week administration were evaluated. For the degrees of amelioration, with respect to a skin lesion at the baseline, the degrees of amelioration in size, color tone, and swelling of skin lesions were evaluated, and at the same time, the degree of amelioration in pain, the degree of amelioration in bleeding, and the degrees of satisfaction of the patients were also evaluated. The evaluation of each item was performed in five ranks (aggravation (20% or more increase in size or height), slight aggravation (less than 20% increase in size or height), no change, slight amelioration (less than 20% reduction in size or height), amelioration (20% or more reduction in size or height)). Photographs before the administration of the sirolimus gel and after the 12-week administration are shown in FIG. 6 to FIG. 10.

In Patient A, although the size was unchanged, the color tone of the hemangioma that had spread from the right cheek to the right periocular region and had spread to the right eyebrow portion, the nasolabial folds and the lower jaw was slightly ameliorated (FIG. 6) . Dark spots on both outer cheek sides are brown pigmented spots.

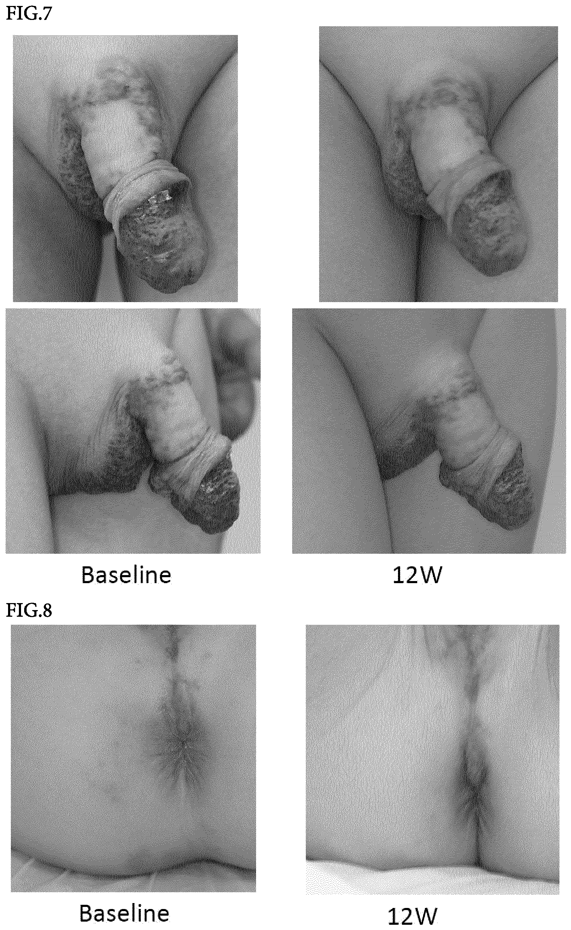

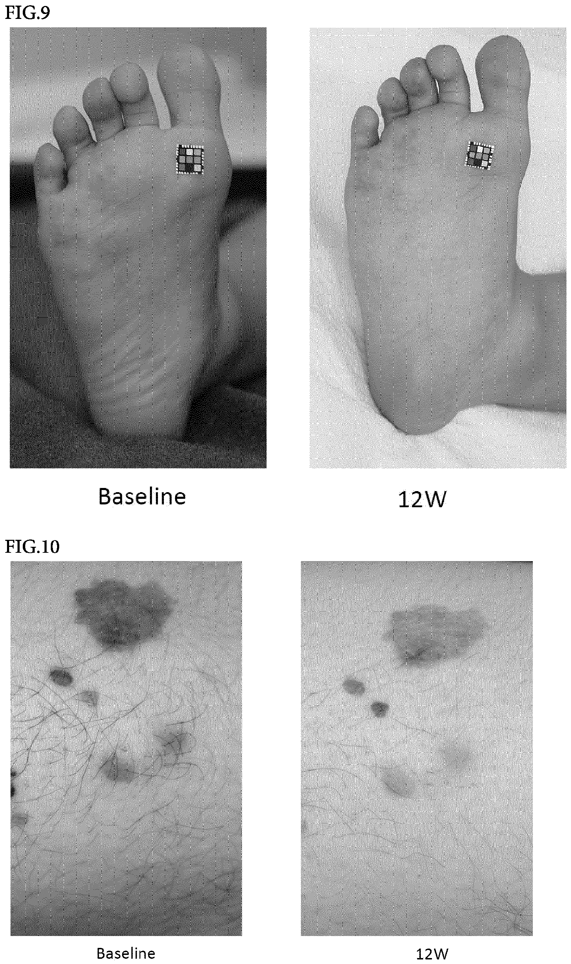

In Patient B, the color of the hemangioma of the penis became pale and its swelling was also ameliorated. As shown in the photographs of FIG. 7, the hemangioma had thickened the glans, body portion, and root portion as compared to those of a normal boy of the same age, and had darkened the color tone of the glans . However, in the photograph after the treatment, the tumor seen on the surface was reduced, and besides, the color of the glans became pale and light, and the whole penis became one size smaller and thinner. This is presumably because the tumor under the skin was reduced and became thin. The hemangioma of the scrotum was also reduced, and became pale in color tone (FIG. 7) . The hemangioma of the right buttock became so pale in color as to be unrecognizable from the skin surface and was reduced in extent (FIG. 8). With regard to the hemangioma of the sole, the prominence of the hemangioma was ameliorated (FIG. 9) . The hemangioma of the lower leg became pale in color and was reduced. Further, Patient B had been complaining of a pain in the lower leg daily, but ceased to complain in the 6th week from the start of topical application.

In Patient C, the color tone of part of the hemangioma became pale, and swelling became flat and was slightly ameliorated. Further, the frequency of bleeding, which had occurred almost daily before topical application, was reduced to about once a week.

Sirolimus was not detected from the blood of any of the patients (measurement limit value: 1.0 ng/mL).

It was revealed from the above-mentioned results that the topical preparation for treating a vascular anomaly of the present invention was safe and exhibited a high therapeutic effect.

Industrial Applicability

The topical preparation for treating a vascular anomaly of the present invention greatly contributes to future medicine.

**Claims**

1. A topical preparation for treating a vascular anomaly, comprising as an active ingredient at least one selected from the group consisting of sirolimus and a sirolimus derivative.

2. The topical preparation according to claim 1, wherein the vascular anomaly is a vascular malformation.

3. The topical preparation according to claim 2, wherein the vascular malformation is a blood vessel malformation and/or a disease including a blood vessel malformation as a pathological condition.

4. The topical preparation according to any one of claims 1 to 3, wherein the sirolimus derivative is at least one selected from the group consisting of everolimus, temsirolimus, ridaforolimus, and zotarolimus.

5. The topical preparation according to any one of claims 1 to 4, wherein the topical preparation is a gel.

6. The topical preparation according to any one of claims 1 to 5, further comprising an alcohol.

7. The topical preparation according to claim 6, wherein the alcohol is ethanol or isopropanol.

8. The topical preparation according to any one of claims 1 to 4, further comprising an ethylene glycol ether.

9. The topical preparation according to any one of claims 1 to 8, wherein a concentration of the active ingredient is from 0.04 wt% to 0.8 wt%.

**Amended claims under Art. 19.1 PCT**

1. (Amended) A topical preparation for treating a blood vessel malformation and/or a disease including a blood vessel malformation as a pathological condition, comprising as an active ingredient at least one selected from the group consisting of sirolimus and a sirolimus derivative.

2. Deleted)

3. Deleted)

4. (Amended) The topical preparation according to claim 1, wherein the sirolimus derivative is at least one selected from the group consisting of everolimus, temsirolimus, ridaforolimus, and zotarolimus.

5. (Amended) The topical preparation according to claims 1 or 4, wherein the topical preparation is a gel.

6. (Amended) The topical preparation according to any one of claims 1, 4 and 5, further comprising an alcohol.

7. (Amended) The topical preparation according to claim 6, wherein the alcohol is ethanol.

8. (Amended) The topical preparation according to claims 1 or 4, further comprising an ethylene glycol ether.

9. (Amended) The topical preparation according to any one of claims 1 and 4 to 8, wherein a concentration of the active ingredient is from 0.04 wt% to 0.8 wt%.

10. Added) The topical preparation according to any one of claims 1 and 4 to 9, wherein the blood vessel malformation is a venous malformation.

**11.** Added) The topical preparation according to any one of claims 1 and 4 to 9, wherein the blood vessel malformation is an arteriovenous malformation.

FIG.1

BEFORE EXTERNAL APPLICATION    AFTER 12-WEEK
EXTERNAL APPLICATION

FIG.2

Cell Proliferation (ISO-HAS-B)

Cell Proliferation (HMVEC)

FIG.3

ISOHAS-B

Ctrl

1 nM

10 nM

100 nM

(48hrs)

FIG.4

HMVEC-d

Ctrl

1 nM

10 nM

100 nM

(96 hrs)

## FIG.5

(a)

normal fibroblast

(b)

baby fibroblast

(c)

HaCaT

(d)

TSC AF fibroblast

(e)

NF1 NF fibroblast

FIG.6

Baseline                    12W

FIG.7

Baseline           12W

FIG.8

Baseline           12W

FIG.9

Baseline                    12W

FIG.10

Baseline                    12W

## INTERNATIONAL SEARCH REPORT

International application No.

PCT/JP2020/005252

### A. CLASSIFICATION OF SUBJECT MATTER

Int.Cl. A61P9/00(2006.01)i, A61P35/00(2006.01)i, A61P43/00(2006.01)i,
A61P17/00(2006.01)i, A61K31/436(2006.01)i, A61K31/662(2006.01)i
FI: A61K31/436, A61P9/00, A61P35/00, A61K31/662, A61P17/00, A61P43/00105
According to International Patent Classification (IPC) or to both national classification and IPC

### B. FIELDS SEARCHED

Minimum documentation searched (classification system followed by classification symbols)
Int.Cl. A61K31/436, A61K31/662, A61P9/00, A61P35/00, A61P43/00, A61P17/00

Documentation searched other than minimum documentation to the extent that such documents are included in the fields searched
Published examined utility model applications of Japan     1922-1996
Published unexamined utility model applications of Japan   1971-2020
Registered utility model specifications of Japan           1996-2020
Published registered utility model applications of Japan   1994-2020

Electronic data base consulted during the international search (name of data base and, where practicable, search terms used)
JSTPlus/JMEDPlus/JST7580(JDreamIII),
CAplus/REGISTRY/MEDLINE/EMBASE/BIOSIS/WPIDS(STN)

### C. DOCUMENTS CONSIDERED TO BE RELEVANT

| Category* | Citation of document, with indication, where appropriate, of the relevant passages | Relevant to claim No. |
|---|---|---|
| X <br> Y | WO 2012/105521 A1 (OSAKA UNIVERSITY) 09.08.2012 (2012-08-09), claims 1, 4, 9, paragraphs [0024], [0036], preparation example 1 | 1-7, 9 <br> 8 |
| X <br> Y | LE SAGE, S. et al., Efficacy and absorption of topical sirolimus for the treatment of vascular anomalies in children: A case series, Pediatric Dermatology, 2018.05.23, vol.35, issue 4, pp. 472-477, DOI:10.1111/pde.13547, abstract, table 1, 3.1 Patient 1 | 1-2, 9 <br> 8 |
| X <br> Y | WO 2018/031789 A1 (THE BOARD OF REGENTS OF THE UNIVERSITY OF TEXAS SYSTEM) 15.02.2018 (2018-02-15), claims 1, 3, 18, paragraphs [0048] | 1-4, 9 <br> 8 |

☒ Further documents are listed in the continuation of Box C.     ☒ See patent family annex.

| | |
|---|---|
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| 06.04.2020 | 21.04.2020 |

| Name and mailing address of the ISA/ | Authorized officer |
|---|---|
| Japan Patent Office <br> 3-4-3, Kasumigaseki, Chiyoda-ku, <br> Tokyo 100-8915, Japan | Telephone No. |

Form PCT/ISA/210 (second sheet) (January 2015)

**INTERNATIONAL SEARCH REPORT**

International application No.

PCT/JP2020/005252

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2015-519399 A (OLATEC INDUSTRIES LLC) 09.07.2015 (2015-07-09), claim 4, paragraph [0025] | 8 |
| Y | JP 2017-529334 A (ABBVIE INC.) 05.10.2017 (2017-10-05), claim 26 | 8 |
| A | JP 2016-175884 A (OSAKA UNIVERSITY) 06.10.2016 (2016-10-06), entire text | 1-9 |
| A | WO 2017/154675 A1 (OSAKA UNIVERSITY) 14.09.2017 (2017-09-14), entire text | 1-9 |
| P, X | CN 109528693 A (WUHAN CONFORM NEW PHARM CO., LTD.) 29.03.2019 (2019-03-29), claims 1, 4, paragraphs [0002]-[0005] | 1-3, 6, 7 |

Form PCT/ISA/210 (second sheet) (January 2015)

**INTERNATIONAL SEARCH REPORT**
Information on patent family members

International application No.

PCT/JP2020/005252

```
WO 2012/105521 A1  09.08.2012   US 2013/0317053 A1
                                claims 1, 4, 9,
                                paragraphs [0065], [0077],
                                preparation example 1
                                EP 2671583 A1

WO 2018/031789 A1  15.02.2018   JP 2019-527711 A
                                claims 1, 3, 18, paragraph [0038]
                                US 2019/0167649 A1
                                EP 3496712 A1
                                KR 10-2019-0039220 A
                                CN 109689053 A

JP 2015-519399 A   09.07.2015   US 2015/0094366 A1
                                claim 4, paragraph [0025]
                                EP 2861228 A1
                                CN 104394860 A

JP 2017-529334 A   05.10.2017   US 2017/0266289 A1
                                claim 26
                                EP 3185852 A1
                                CN 106794126 A

JP 2016-175884 A   06.10.2016   US 2018/0110759 A1
                                whole document
                                EP 3275441 A1
                                CN 107708694 A

WO 2017/154675 A1  14.09.2017   EP 3427733 A1
                                whole document
                                CN 108697691 A

CN 109528693 A     29.03.2019   (Family: none)
```

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012105521 A1
- WO 2015199248 A1
- WO 2017154675 A1
- WO 2016152519 A1

**Non-patent literature cited in the description**

- New Cultured Cell Experimental Methods. Molecular Biology Research
- **JIKKEN IGAKU.** BioManual UP Series. Yodosha Co., Ltd,